# EUROPÄISCHE PATENTSCHRIFT

(11) **EP 0 616 295 B1**
(45) Veröffentlichungstag und Bekanntmachung des Hinweises auf die Patenterteilung: **31.03.1999**
(21) Anmeldenummer: 93111959.8
(22) Anmeldetag: 27.07.1993
(51) Int. Cl.: G06K 9/00, A61B 5/117

(54) **Verfahren und Anordnung zur Verifikation von Fingerabdrücken**
Method and arrangement for finger-print verification
Procédé et arrangement de vérification des empreintes digitales

(30) Priorität: 16.02.1993 DE 4304605
(43) Veröffentlichungstag der Anmeldung: 21.09.1994
(73) Patentinhaber: HAHN BIOMETRIX GmbH i. G., D-70731 Fellbach (DE)
(72) Erfinder: Nguyen, Xuan Phuc, D-68163 Mannheim (DE); Ulpins, Waldemar, D-68309 Mannheim (DE); Schönig, Christian, D-67433 Neustadt/Weinstrasse (DE); Wirnitzer, Bernhard, D-69502 Hemsbach/Bergstrasse (DE); Abel, Konrad, D-68775 Ketsch/Rhein (DE)
(74) Vertreter: Muschka, Wilhelm, Dipl.-Ing.

(56) Entgegenhaltungen:
- US-A- 4 120 585
- US-A- 4 394 773
- PATENT ABSTRACTS OF JAPAN vol. 8, no. 51 (P-259) 8. März 1984 & JP-A-58 201 178 (NIPPON DENSHIN DENWA KOSHA) 22. November 1983
- PATENT ABSTRACTS OF JAPAN vol. 12, no. 300 (P-745) 16. August 1988 & JP-A-63 074 026 (FUJITSU LTD) 4. April 1988

## Beschreibung

Die Erfindung bezieht sich auf ein Verfahren zur Verifikation von Fingerabdrücken sowie auf eine Anordnung zur Durchführung dieses Verfahrens.

Ein solches Verfahren ist z.ß. aus der Patentanmeldung Akz. P 34 21 220.5-51 bekannt: Zwischen Reflexionsprisma und bildseitigem Endabschnitt sind zwei Ablenkprismen angeordnet, von denen das eine der anamorphotischen Vergrößerung und das andere dem Ausgleich des Astigmatismus dient. Dabei wird die perspektivische Gesetzmäßigkeit ausgenutzt, daß Ablenkprismen in spezieller Kombination eine anamorphotische Vergrößerung ohne Astigmatismus bewirken. Die der Anamorphose eigene optische Verzerrung läßt sich mittels eines Ablenkprismas mit bestimmtem Phasenwinkel beheben, während der Astigmatismus mittels eines hiervon abgesetzten Prismas mit erheblich geringerem Prismenwinkel ausgeglichen wird. Von Nachteil ist hierbei die ganz spezielle Prismenanordnung, die außerdem durch die gegenseitige Beabstandung vergleichsweise platzaufwendig ist. Außerdem wird nur der optische Aufnahmeteil behandelt, während Hinweise auf das Prinzip des Auswerteverfahrens fehlen.

Ein ähnliches Verfahren behandelt auch die EP mit Veröffentlichungs-Nr. 88308416.2: Das Prisma, auf dessen einer Seite der Finger aufliegt, wird von einer anderen Seite aus mit monochromatischem Licht von LEDs über Kondensorlinse und optischen Diffusor beleuchtet, so daß in den Vertiefungen des Fingers eine Totalreflexion entsteht, während eine solche an den Erhebungen verhindert wird. Die Abbildungsoptik projiziert das Bild auf eine CCD-Kamera, wobei das Korrekturprisma in funktioneller Verbindung mit dem Auflageprisma die anamorphotische Vergrößerung bewirkt. Auch hier wird lediglich der optische Aufnahmeteil behandelt, wohingegen Hinweise auf die Verarbeitung fehlen.

Vergleichbare Verfahren sind ferner den Fachzeitschriften Seiko lgaki Holografic Fingerprint Sensor 1989 Fingitsu Sci. Techn. I. Seiten 287-296 und Philips Techn. Rundschau 25, 1984 zu entnehmen. Auch hier wieder der bekannte physikalische Effekt der Totalreflexion und seine Aufhebbarkeit beim Auflegen des Fingers auf eine Seite eines beleuchteten Prismas und die Abbildung des mit hohem Kontrast erzeugten Fingerabdrucks über ein Abbildungsobjektiv auf eine CCD- oder Videokamera, während nähere Hinweise auf die Verarbeitung nicht vorhanden sind.

Schließlich gibt es auch noch eine Fingerabdruckidentifikationsmethode, die z.B. in der EP 00 90 377 B1 oder der EP mit der Veröffentlichungs-Nr. 83 102 997.0 behandelt wird. Hier werden in einem Binärbild Anomalien eines Fingerabdrucks mit vorgegebenen Gruppen von Schablonen verglichen. Zu letzteren zählen Anfänge, Enden, Verzweigungen, Überkreuzungen, Berührungen und Unterbrechungen von Linien sowie Brücken, Poren, Ortsfrequenzen u.a. Derartige Verfahren der Merkmalextraktion erfordern aber eine rechenaufwendige und zeitraubende Vorverarbeitung wie Filterung oder Skelettierung. Auch Restaurationsverfahren oder Verfahren zur Unterdrückung von Poren können erforderlich sein.

Außerdem ist aus Patent Abstracts of Japan, vol. 8, n° 51 (P- 259) 8.3.84 & JP- A- 58201178 ein Verfahren zur Verifikation von Fingerabdrucken bekannt, bei dem ein Finger auf ein von einer Lichtquelle bestrahltes, reflektierendes Prisma gelegt wird, und
a) die Bildaufnahme über den Anpreßdruck gesteuert wird;
b) der richtige Anpreßdruck mittels einer Kontrollanzeige überwacht wird und
c) außer der Auswertung auch noch Anpreßdruck und Identifikation von ein und derselben Signalprozessoreinheit sequentiell übernommen werden.

Die Aufgabe der Erfindung wird in einer Verbesserung der Erkennsicherheit des gattungsgemäßen Verfahrens bei gleichzeitig geringerem Hardwareaufwand gesehen. Diese Aufgabe wird erfindungsgemäß durch die Kennzeichnungsmerkmale des Patentanspruchs 1 gelöst. Der breite Einsatz der Signalprozessoreinheit gewährleistet im Hinblick auf die Kürze der Verarbeitungszeit neue Verfahren der Bildaufnahme und eine geschickte Merkmalsextraktion, die keine aufwendige Vorverarbeitung, das heißt weder eine totale Entzerrung noch eine Beseitigung von Astigmatismus benötigt. Darüber hinaus erhält man auf diese Weise optimale, reproduzierbare Fingerabdruckbilder.

Im folgenden wird an Hand einer Zeichnung ein Ausführungsbeispiel der Erfindung erläutert, wobei die in den einzelnen Figuren einander entsprechenden Teile dieselben Bezugszeichen aufweisen. es zeigt
- Fig. 1: die für den vorgesehenen Verwendungszweck für sich genommen bekannte Prismenkombination,
- Fig. 2: eine Prinzipskizze der erfindungsgemäßen Anordnung einschließlich Prismenkombination und Druckaufnehmer,
- Fig. 3a: einen Fingerabdruck in vergrößertem Maßstab mit darauf vorgesehenen Zeilenmarkierungen mit Lauflängencode,
- Fig. 3b: die unterschiedlichen Lauflängen in den einzelnen Zeilen und
- Fig. 4: die Kombination verschiedener Markierungslinien.

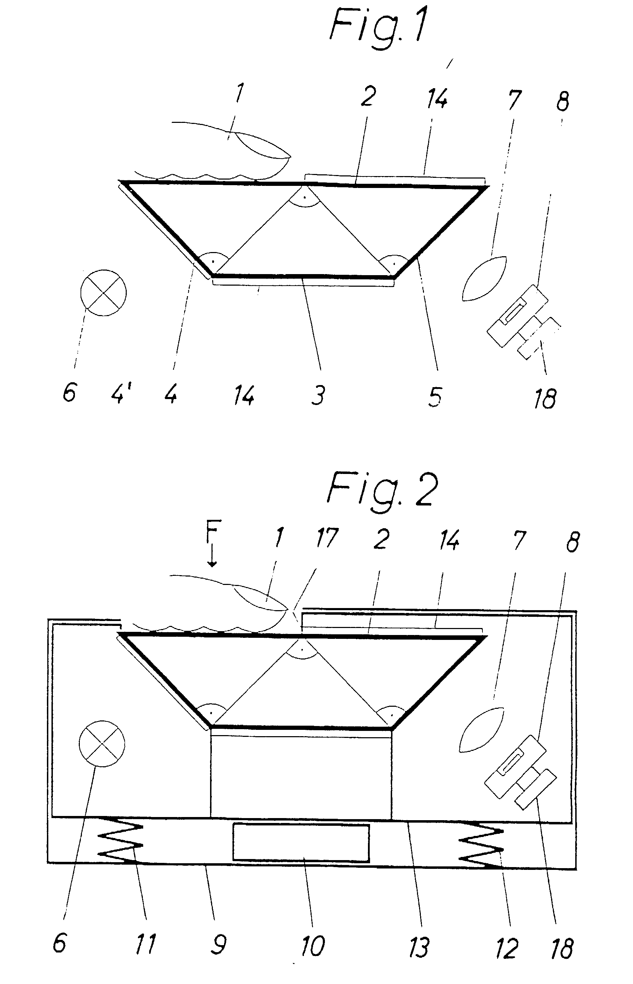

In Fig. 1 liegt der Finger 1 einer menschlichen Hand auf der der beiden parallelen Ebenen 2 und 3 der Prismenkombis 5. Im Bereich vor der - in Blickrichtung - linSeitenfläche 4 ist im vorliegenden AusführungsbeiBeleuchtung 6 installiert, die ein kontrastreiches Fingers bewirken soll. Oberhalb der Seitenfläche 4 ist der 4', eine Mattscheibe, angebracht. Im Bereich der liegenden anderen kurzen Seitenfläche 5 befindet sich der Bildaufnahmesensor 8 mit der ihm funktionell vorgeschalteten Abbildungsoptik 7. Hierbei schließt die Bildebene des Bildaufnahmesensors mit dem auf der Ebene 2 aufliegenden Finger 1 einen schiefen Winkel ein. Oberhalb der Ebenen 2 und 3 ist auch noch je eine Reflexionsscheibe 14 mit definierter Absorption vorgesehen. Bei einem anderen Ausführungsbeispiel der Erfindung läßt sich die Zuordnung der verschiedenen Funktionskomponenten zu den einzelnen Ebenen bzw. Flächen er Prismenkombination 2 bis 5 auch variieren, ohne daß dadurch der Rahmen der Erfindung verlassen würde.

Die nach erfolgter Abbildung erforderlichen Verarbeitungsschritte vereinfachen sich, wenn das Bild der Einlernphase dem Bild der Verifikationsphase möglichst nahekommt. Eine Vielzahl von Störungseinflüssen sind hierbei denkbar, die zu unerwünschten Unterschieden zwischen dem eingelernten und dem zu überprüfenden Fingerabdruckbild 15 führen. Die erwähnte Nähe der spezifischen Eigenschaften von zum Vergleich anstehenden Bildern erhält man, sofern die Bildaufnahme beim Ein lernen und beim Überprüfen unter gleichem Anpreßdruck des Fingers 1 erfolgt. Eine weitere Verbesserung läßt sich dadurch erzielen, daß beim Einlernen Bilder mit unterschiedlichen Anpreßdrücken aufgenommen werden und die Bildqualität jeweils automatisch beurteilt wird. Eines der möglichen Kriterien für die Bildqualität ist z.B., ob und in welchem Ausmaß die Breite der dunklen und der hellen Linien eines Fingerabdruckbildes 15 übereinstimmt. Man wählt dann denjenigen Anpreßdruck, bei dem die Bildqualität optimal ist und löst für den Vorgang der Verifikation die Bildaufnahme beim gleichen Anpreßdruck aus.

Um den Anpreßdruck zu messen, wird die Prismenkombination 2 bis 5 gemäß Fig. 2 mit einem Druckaufnehmer 9 bis 13 konstruktiv verbunden. Hierfür ist in dem Gehäuse 9 bodenseitig ein Druckmesser 10 vorgesehen, auf dem über die Federn 11 und 12 die in der Fassung 13 - in Richtung des Pfeils F - beweglich gehalterte Prismenkombination aufliegt. An seiner Oberseite besitzt das Gehäuse eine Ausnehmung 17, durch die hindurch der zu verifizierende Finger 1 auf die Ebene 2 der Prismenkombination 2 bis 5 gelegt wird, um den durch den Pfeil symbolisierten Anpreßdruck F zu bewerkstelligen. Bei einem anderen, zeichnerisch nicht dargestellten Ausführungsbeispiel ist es selbstverständlich auch denkbar, die Federn 11 und 12 durch Piezodruckaufnehmer oder mit Dehnungsmeßstreifen kombinierte Gummiaufleger zu ersetzen.

Sobald nun der Anpreßdruck auf die Prismenkombination 2 bis 5 den verlangten Wert erreicht hat, erfolgt die Bildaufnahme mit Hilfe des Bildaufnehmers 8, der sich z.B. aus CCD-Bildsensoren zusammensetzt, wobei Anfangs- und Endzeitpunkt der Belichtung durch elektronische Steuersignale wählbar sind. im Fall kompletter CCD-Kameras mit Video-Norm-Signalen erfolgt die Bildaufnahme in festen Zeitrastern unter Verwendung einer Blitzlichtbeleuchtung.

Eine Verfeinerung der vorbeschriebenen Methode läßt sich erreichen, wenn man für die Rand- und die zentralen Zonen des Fingerabdrucks 15 den optimalen Anpreßdruck getrennt ermittelt sowie Bildaufnahme und Auswertung für jede Zone getrennt durchführt. Bei dieserart Bildaufnahme ergeben sich für die nachfolgende Bildauswertung und Identifikation ein vergleichsweise geringer Rechenaufwand. Mit nur einer preiswerten Signalprozessoreinheit 18 lassen sich sämtliche Auswertungen in weniger als einer Sekunde durchführen, eingeschlossen Drucksteuerung, Bildaufnahme und Auslesen des Bildaufnahmesensors. Sofern eine erhöhte Erkennsicherheit erforderlich ist, kann die Signalprozessoreinheit zusätzlich auch noch eine Sprecherkennung durchführen.

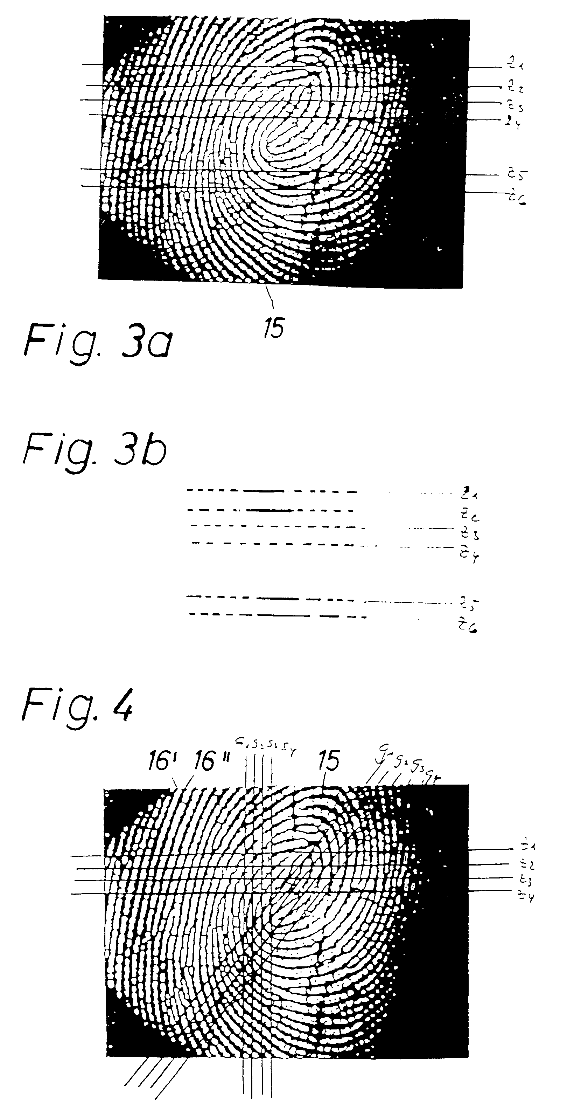

Beim ersten Schritt der Verarbeitung werden die Bildintensitäten mit einem Schwellwert verglichen. Intensitäten, die größer als der Schwellwert sind, werden auf EINS und solche, die kleiner als der Schwellwert sind, auf NULL gesetzt. Von dem auf diese Weise erhaltenen Binärwert interessieren die Bildkoordinaten, an denen ein Übergang von NULL auf EINS oder von EINS auf NULL erfolgt. Gemäß der Erfindung und Fig. 4 werden nun aus diesen Koordinaten die Lauflängen der hellen Bereiche des Fingerabdrucks 15 bzw. der Zwischenräume zwischen den einzelnen Fingerabdrucklinien 16^{I} bis 16ⁿ in Zeilenrichtung Z₁ bis Zₙ, in Spaltenrichtung S₁ bis Sₙ oder in Linien G₁ bis Gₙ unter einem beliebigen Winkel ermittelt. Dazu werden auf einer Zeile lediglich Anfang und Ende eines hellen Bereiches registriert (Fig. 3b). Diese Auswertung kann in Echtzeit, das heißt im Pixeltakt erfolgen.

Diejenige Signalprozessoreinheit, die den Bildaufnahmesensor 8 ausliest, kann gleichzeitig für den Schwellwertvergleich und die Lauflängenkodierung herangezogen werden, so daß kein digitaler Bildspeicher zum Ablegen des Fingerabdruckbildes 15 benötigt wird. Außerdem wird mit Hilfe von nur geringem Rechenaufwand die Auswertung der Lauflängen durch die Kombination mit anpreßgesteuerter Bildaufnahme bewerkstelligt.

In Fig. 3b wurden aus der Menge aller Zeilen Z₁ bis Zₙ einige typische Zeilen, nämlich die Zeilen Z₁ bis Z₆, ausgewählt und die zugehörigen Lauflängen markiert. Diese Lauflängen bilden für jede dieser Zeilen einen ganz speziellen Code - den Lauflängencode. Es ist nun während der Bildaufnahme möglich, die Lauflängen aller Zeilen eines Fingerprints, das heißt also die tatsächliche Echtzeit,zu ermitteln. Als trennungswirksamer Merkmalsatz zur Identifikation werden entweder die Lauflängencodes aller Zeilen Z₁ bis Zₙ benutzt oder aber es werden spezielle Lauflängenkonfigurationen ausgewählt. Mögliche Kriterien hierfür sind entweder die Lage von n Zeilen mit größten Lauflängen, die Lage von n Zeilen mit größter Lauflängenvariation oder die Lage von n Zeilen mit geringster Lauflängenvariation und beliebiger Kombination, wobei n eine beliebige Zahl sein kann.

Die Identifizierung erfolgt nun über eine Korrelation der Lauflängencodes aller Zeilen Z₁ bis Zₙ oder der speziell markierten Zeilen Z₁ bis Z₆ mit dem in einem Speichermedium abgespeicherten Merkmal satz des zu identifizierenden Fingerabdrucks 15. Wie Fig. 4 zeigt, kann aber auch die Lage der markierten Zeilen bzw. ihre Kombination zum Vergleich herangezogen werden. Selbst Spalten S₁ bis Sₙ, unter einem bestimmten Winkel angeordnete Linien G₁ bis Gₙ oder die Kombination einer beliebig ausgewählten Zahl von Zeilen, Spalten und winkelig dazu verlaufenden Linien lassen sich zur Merkmalsextraktion und Identifikation auswerten.

Translationsinvariant, das heißt unabhängig von der translatorischen Verschiebung des Fingerabdrucks 15 auf die Prismenkombination 2 bis 5, wird der Identifizierungsvorgang, wenn als Merkmalsatz eine ausgewählte Kombination von markierten Lauf längen benutzt wird und nicht etwa die Lauflängencodes von festen Ortskoordinaten.

## Patentansprüche

1. Verfahren zur Verifikation von Fingerabdrücken, bei dem ein Finger auf die Bildebene eines Signale weiterleitenden optischen Bauteils gelegt wird, der Anpreßdruck ermittelt wird, die fingerspezifischen Merkmale zumindest für einzelne Randund / oder zentrale Zonen getrennt oder gleichzeitig mittels Lichtquelle auf einem Bildaufnahmesensor abgebildet werden, wobei die Bildaufnahme über den Anpreßdruck, einen Schwellenwertvergleich und eine Kontrollanzeige gesteuert wird und schließlich eine Auswerteeinheit die Identifikation ermöglicht, und bei dem :
a) Anpreßdruck und Bildaufnahme von ein und derselben Signalprozessoreinheit (18) gesteuert werden,
b) die Bildaufnahme bei dem Anpreßdruck erfolgt, bei dem dies Breite der dunklen und hellen Linien des Fingerabdrucks innerhalb einer vorgegebenen Toleranz liegen und diese Auswertung vom gleichen Signalprozessor durchgeführt wird und
c) der gleiche Signalprozessor auch beim Auslesen des Bildes die Bildintensitäten mit dem Schwellenwert vergleicht und lediglich Anfang und Ende der hellen Bereiche - mit einer Intensität oberhalb der Schwelle - im Pixeltakt registriert werden.

2. Verfahren nach Anspruch 1, **dadurch gekennzeichnet,** daß aus den Bildkoordinaten die Lauflängen der hellen Bereiche des Fingerabdrucks (15) in Zeilenrichtung (Z₁ bis Zₙ), in Spaltenrichtung (S₁ bis Sₙ) oder in Linien (G₁ bis Gₙ) unter einem beliebigen Winkel ermittelt werden (Fig. 4).

3. Verfahren nach Anspruch 2, **dadurch gekennzeichnet,** daß auf einer Zeile (Z₁ bis Zₙ) lediglich Anfang und Ende eines hellen Bereiches - z.B. im Pixeltakt - registriert werden.

4. Verfahren nach einem der vorausgehenden Ansprüche, **dadurch gekennzeichnet,** daß bei einem bestimmten Anpreßdruck und der Verwendung von CCD-Kameras mit Video-Norm eine Blitzlichtbeleuchtung gezündet wird.

5. Anordnung zur Durchführung des Verfahrens nach Patentanspruch 1 oder einem der folgenden, unter-Verwendung eines Prismas, auf das der interessierende Finger aufgelegt wird, vom Prisma betätigten Druckaufnehmern und einer Kontrollanzeige zum überwachen des Anpreßdrucks sowie einer Lichtquelle, mit deren Hilfe die fingerspezifischen Signale auf einem Bildaufnahmesensor abgebildet werden, bei der :
a) anstelle des Prismas eine als Doveprisma ausgebildete Prismenkombination (2 bis 5) vorgesehen ist,
b) oberhalb der einen kurzen Seitenfläche (4) des Doveprismas ein Diffusor (4') mit funktionell vorgeschalteter Lichtquelle (6) und im Bereich der gegenüberliegenden anderen kurzen Seitenfläche (5) der Bildaufnahmesensor (8) mit der ihm funktionell vorgeschalteten Abbildungsoptik (7) vorgesehen sind,
c) die zueinander parallelen Seiten des Doveprismas wenigstens teilweise mit je einer Reflexionsscheibe (14) mit definierter Absorption abgedeckt sind und
d) in dem die Anordnung umfassenden Gehäuse (9) oberseitig eine der Auflage des Fingers (1) angepaßte Ausnehmung (17) vorgesehen ist.

## Claims

1. A method for the verification of finger prints, in which a finger is placed on the image plane of a signal-relaying optical component, the application pressure is determined, and the finger-specific features are imaged on an image recording sensor at least for individual edge regions and/or central regions separately or at the same time by means of a light source, wherein the image recording is controlled via the application pressure, a threshold value comparison and a control indication and, finally, an analysis unit enables identification, and wherein:
a) application pressure and image recording are controlled by one and the same signal processor unit (18),
b) the image recording is carried out at the application pressure at which the width of the dark and light lines of the finger print lie within a predetermined tolerance and the analysis is carried out by the same signal processor and
c) the same signal processor additionally compares the image intensities with the threshold value when the image is read out and only the beginning and end of the light regions - having an intensity above the threshold - are recorded in the pixel cycle.

2. A method according to claim 1, characterised in that the extents of the light regions of the finger print (15) are determined from the image co-ordinates in a row direction (Z₁ to Zₙ), in a columnar direction (S₁ to Sₙ) or in lines (G₁ to Gₙ) at any angle (Fig. 4).

3. A method according to claim 2, characterised in that in a row (Z₁ to Zₙ) only the beginning and end of a light region - for example, in the pixel cycle - are recorded.

4. A method according to any one of the preceding claims, characterised in that a flash exposure is triggered at a specific application pressure when video-standard CCD cameras are used.

5. An arrangement for carrying out the method according to claim 1 or any one of the subsequent claims, using a prism onto which the finger in question is placed, pressure sensors activated by the prism, a control indication for monitoring the application pressure, and a light source by means of which the finger-specific signals are imaged on an image recording sensor, wherein:
a) a prism combination (2 to 5) in the form of a dovetail prism is provided in place of the prism,
b) above a first short lateral face (4) of the dovetail prism, a diffuser (4') is provided with a light source (6) which is connected upstream in terms of function and, in the region of the opposing second short lateral face (5), the image recording sensor (8) is provided with the optical imaging unit (7) which is connected upstream thereof in terms of function,
c) the mutually parallel sides of the dovetail prisms are each covered, at least partially, with a reflective plate (14) having defined absorption, and
d) a recess (17) adapted for placing the finger (1) is provided in the top of the housing (9) surrounding the arrangement.

## Revendications

1. Procédé pour vérifier des empreintes digitales, selon lequel on place un doigt sur le plan image d'un composant optique transmettant des signaux, on détermine la pression d'application, et on forme l'image des caractéristiques spécifiques au doigt, au moins pour des zones marginales séparées et/ou pour des zones centrales séparément ou simultanément, à l'aide d'une source de lumière, sur un capteur d'enregistrement d'images, l'enregistrement de l'image étant commandé par l'intermédiaire de la pression d'application, d'une comparaison à une valeur de seuil et d'un affichage de contrôle, tandis que finalement une unité d'exploitation permet l'identification, et selon lequel :
a) la pression d'application et l'enregistrement de l'image sont commandés par une même unité (18) formant processeur de signaux,
b) l'enregistrement de l'image s'effectue alors que la pression d'application est telle que la largeur des traits sombres et des traits clairs de l'empreinte digitale étant située à l'intérieur d'une tolérance prédéterminée, et cette évaluation étant exécutée par le même processeur de signaux, et
c) le même processeur de signaux compare les intensités de l'image à la valeur de seuil également lors de la lecture de l'image, et seuls le début et la fin des zones claires - ayant une intensité supérieure au seuil - sont enregistrés à la cadence des pixels.

2. Procédé selon la revendication 1, caractérisé en ce que les longueurs de projection des parties claires de l'empreinte digitale (15) sont déterminées (figure 4) à partir des coordonnées d'image, dans la direction des lignes (Z₁ à Zₙ), dans la direction des colonnes (S₁ à Sₙ) ou suivant des lignes (G₁ à Gₙ) disposées sous un angle quelconque.

3. Procédé selon la revendication 2, caractérisé en ce que seuls le début et la fin d'une zone claire sont enregistrés - par exemple à la cadence des pixels - sur une ligne (Z₁ à Zₙ).

4. Procédé selon l'une des revendications précédentes, caractérisé en ce que pour une pression d'application déterminée et dans le cas de l'utilisation de caméras à dispositif CCD ayant une norme vidéo, un éclairage flash est amorcé.

5. Dispositif pour la mise en oeuvre du procédé selon la revendication 1 ou l'une des suivantes, moyennant l'utilisation d'un prisme sur lequel le doigt auquel on s'intéresse est appliqué, de capteurs de pression actionnés par le prisme, et d'un dispositif d'affichage de contrôle pour contrôler la pression d'application, ainsi que d'une source de lumière à l'aide de laquelle les signaux spécifiques au doigt sont formés sur un capteur d'enregistrement d'images, dans lequel :
a) à la place du prisme il est prévu un ensemble combiné de prismes (2 à 5) agencé sous la forme d'un prisme de Dove,
b) un diffuseur (4'), équipé d'une source de lumière (6) branchée fonctionnellement en avant de ce diffuseur, est prévu au-dessus d'une courte surface latérale (4) du prisme de Dove, et le capteur d'enregistrement d'images (8), en amont duquel est branché du point de vue fonctionnel un système optique de formation d'images (7), est prévu dans la zone de l'autre courte surface latérale opposée (5) du capteur d'enregistrement d'images (8),
c) les faces parallèles du prisme de Dove sont recouvertes au moins en partie par une plaque réfléchissante respective (14) produisant une absorption définie, et
d) un évidement (17) adapté pour l'application du doigt (1) est prévu sur le côté supérieur, dans le boîtier (9) entourant le dispositif.
